# EUROPEAN PATENT APPLICATION

(11) **EP 2 455 487 A2**
(43) Date of publication of application: **23.05.2012**
(21) Application number: 10800041.5
(22) Date of filing: 15.07.2010
(51) Int. Cl.: C12Q 1/68, G01N 33/50, G01N 30/90

(54) **CHROMATOGRAPHIC SYSTEM FOR NUCLEIC ACID DETECTION**

(30) Priority: 17.07.2009 KR 20090065275
(71) Applicant: Boditech Med Inc., Gangwon-do 200-883 (KR)
(72) Inventor: CHOI, Eui-yul, Chuncheon-si Gangwon-do 200-770 (KR); NAHM, Kie-bong, Seoul 138-220 (KR); JEONG, Dong-seok, Chuncheon-si Gangwon-do 200-936 (KR); KIM, Sung-joong, Chuncheon-si Gangwon-do 200-060 (KR)
(74) Representative: Maiwald Patentanwalts GmbH
(86) International application number: PCT/KR2010/004598
(87) International publication number: WO 2011/008030

(57) **Abstract**

The present disclosure provides a strip for separation and sequence specific detection of nucleic acids in a sample, a system for sequence specific detection of nucleic acids comprising the strip of the present disclosure and an epifluorescence detection device, and a method for qualitative and/or quantitative determination of a nucleic acid using the strip. The strip, system and method of the present disclosure is easy to use and provides accurate and reliable results due to its high sensitivity and specificity in a relatively short analysis time compared to the conventional assays.

## Description

### CROSS-REFEREBCE TO REKATED APPLICATIONS

This application is claiming priority of PCT/KR2010/004598 entitled "Chromatographic System for Nucleic Acid Detection", filed on July 15, 2010, which claims priority of Korea Patent Application bearing serial number 10-2009-0065275, filed on July 17, 2009, the entire contents of which are hereby incorporated by reference.

### FIELD OF THE INVENTION

The present disclosure generally relates to nucleic acid detection area. Particularly, the present disclosure relates to a strip, a method and a system based on a lateral flow chromatographic separation and sequence specific detection of nucleic acids in a sample.

### BACKGROUND OF INVENTION

Research area and industrial field where detection of nucleic acids are inevitable, whether the detection relates to the determination of presence or absence of nucleic acids of interest, or to the quantitative or qualitative measurement of nucleic acids, generally use methods utilizing the sequence specific hybridization of nucleic acids. One of such methods is polymerase chain reaction based assay where a particular sequence of interest is selectively amplified and the amplified products are then analyzed by gel electrophoresis which is then visualized by sequenced specific hybridization methods such as southern or northern blot or by staining with dye.

However, such methods are a complex process which requires long hours of works not only for the sample preparation and reaction but also for the visualization. Further the reaction results are not very sensitive requiring large amount of input material and tend to be very sensitive to various reaction conditions such as hybridization time or temperature, and the reaction buffer or primer used and the like. Thus, to set up an optimal conditions to obtain a reliable result, repetitive experiments by highly skilled technicians are required.

Nucleic acid detections are widely used for clinical uses such as diagnosis or screening of a disease or prognosis of a therapy. Therefore, there are demands for simple and convenient, yet reliable systems or products or methods for detecting nucleic acids in a sample.

### SUMMARY OF THE INVENTION

The present disclosure relates to a strip, a system and a method for nucleic acid detection and/or quantification which are capable determining the presence, absence and/or an amount of a particular nucleic acid sequence in a sample. The present disclosure is based on a lateral flow chromatographic separation and sequence specific detection of nucleic acids in a sample, which is easy to use and provides accurate and reliable results in a relatively short analysis time due to its high sensitivity and specificity compared to the conventional systems.

In one aspect, the present disclosure relates to a lateral flow assay strip for qualitative and/or quantitative determination by sequence specific detection of an analyte, i.e., nucleic acid molecules in a sample. The strip of the present disclosure comprises a sample pad providing a site of sample application; an absorption pad; a chromatographic medium located therebetween in a way to allow for capillary flow communication with each other, the chromatographic medium containing at least one kind of capture molecules which are capable of hybridizing sequence specifically to a particular nucleic acid sequence in a sample which is applied to the sample pad; and a solid support backing the chromatographic medium, the sample pad and the absorption pad which are located on the solid support in the same plane so as to permit capillary flow communication with each other. In one embodiment, the solid support has two ends with the sample pad and the absorption pad, each being located at one end of the support, respectively. In other embodiment, the at least one capture molecules are immobilized at a particular location of known on the chromatographic medium.

In other aspect, the present invention relates to a lateral flow qualitative and/or quantitative assay strip for sequence specific detection of analyte, i.e., nucleic acids in a sample. The strip of the present disclosure comprises a sample pad providing a site of sample application; an absorption pad; a chromatographic medium located therebetween in a way to allow capillary flow communication with each other, the chromatographic medium containing at least one kind of capture molecules wherein the capture molecules are capable of hybridizing sequence specifically to a particular nucleic acid sequence in a sample which is applied to the sample pad and is labeled with a fluorescent material or biotin and is a product of polymerase chain reaction; and a solid support backing the chromatographic medium, the sample pad and the absorption pad which are located on the solid support in the same plane so as to permit capillary flow communication with each other. In one embodiment, the solid support has two ends with the sample pad and the absorption pad, each being located at one end of the solid support, respectively.

In still other aspect, the present disclosure provides the strip as described above wherein the capture molecules have a nucleic acid sequence derived from Human Papilloma virus (HPV).

In still another aspect, the present disclosure provides the strip as described above which are capable of detecting and differentiating different sub-types of HPV selected from the group consisting of 16, 18, 26, 31, 33, 35, 39, 45, 51, 52, 53, 56, 58, 59, 66, 68, 70, 73, 82, 6, 11, 40, 42, 43, 44, 54, 61, 72, 81 and CP108.

In still other aspect, the present disclosure provides a system for detecting a nucleic acid in a sample comprising a strip in accordance with the present disclosure and an epifluorescence detection device for detecting signals from a capture molecule in any one of the strip of the present disclosure as described above. In one embodiment, the epifluorescence is laser-induced.
1. In still other aspect, the present disclosure provides a method of qualitative and/or quantitative determination of nucleic acids in a sample using any one of the strips in accordance with the present disclosure as described above, comprising the steps of applying a liquid sample containing a nucleic acid sequence(s) of interest labeled with a fluorescent or a biotin to a sample pad of the strip of the present disclosure; and allowing the liquid sample to flow to the absorption pad of the strip, wherein the capture on a particular location of the strip react sequence-specifically with the nucleic acid sequences of interest to form a hybrid ; and detecting a signal emitted from the labels on the hybrid using a epifluorescence detection device, where the presence of the signal indicates the presence of the nucleic acid of interest or the signal is used to quantify the amount of the nucleic acid of interest in the sample.

In one embodiment, the capture molecules have a nucleic acid sequence derived from Human Papilloma virus (HPV).

In still another aspect, the present disclosure provides the methods as described above which are capable of detecting and differentiating different sub-types of HPV selected from the group consisting of 16, 18, 26, 31, 33, 35, 39, 45, 51, 52, 53, 56, 58, 59, 66, 68, 70, 73, 82, 6, 11, 40, 42, 43, 44, 54, 61, 72, 81 and CP108.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

A more complete appreciation of the invention, and many of the attendant advantages thereof, will be readily apparent as the same becomes better understood by reference to the following detailed description when considered in conjunction with the accompanying drawing, wherein:

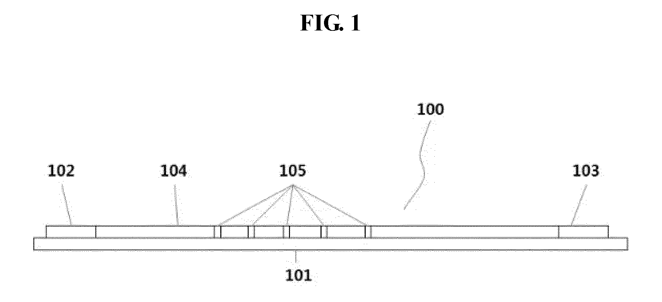

FIG 1 is a schematic view of an exemplary embodiment of a strip in accordance with the present disclosure. Reference characters indicate: 101: Lateral flow strip; 102: Sample pad; 103: Absorption pad; 104: Chromatographic medium; and 105: Signal detection area (also called analyte measurement area).

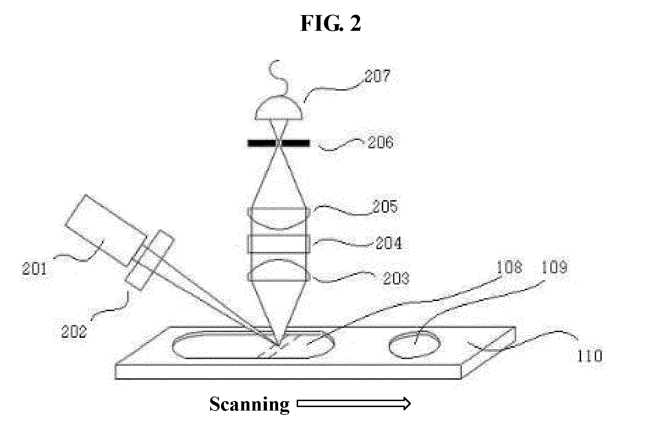

FIG 2 is a schematic view of a laser-induced epifluorescence detection device. Reference characters indicate: 108: Test window; 109: Sample application site; 110: Housing for strip; 200: Laser induced epifluorescence detection device; 201: Laser; 202: Lens for laser beam control; 203: Collector lens; 204: Fluorescent filter; 205: Condensing lens; 206: Spatial filter; and 207: Light detector.

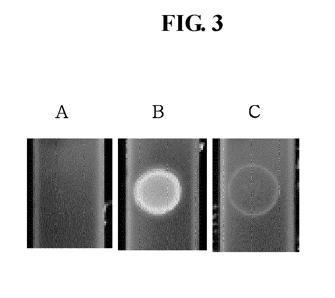

FIG 3 is the results of a DNA immobilization assay to optimize the condition for immobilization onto a nitrocellulose membrane. (a) without UV irradiation; (b) one hour drying of the membrane followed UV irradiation; (c) UV irradiation without drying the membrane. The area with yellow color indicates the immobilized DNA.

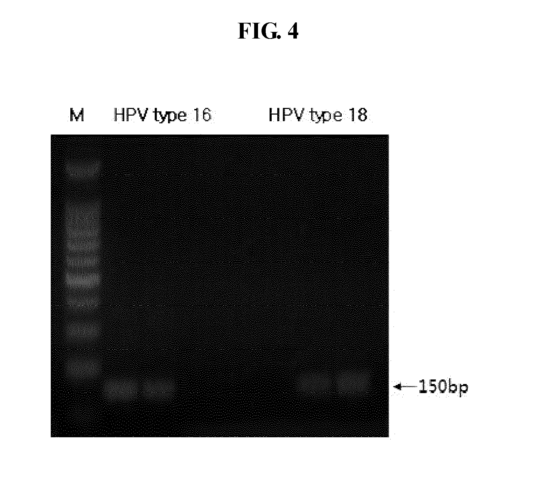

FIG 4 is the electrophoresis result run on a 2% agarose gel of PCR amplified products using the primers specific for HPV 16 and 18, respectively, and labeled with Cy5. Red indicates the presence of DNA and M indicates the size marker.

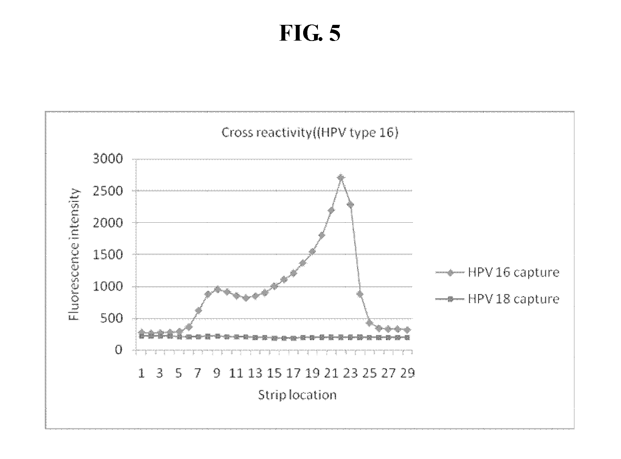

FIG 5 is the results of an assay to determine the cross reactivity among nucleic acid sequences detecting different subtypes of HPV HPV 16 in a sample was detected using a system in accordance with an embodiment with the present disclosure where the DNAs specific for HPV 16 and 18 were used as the capture molecules.

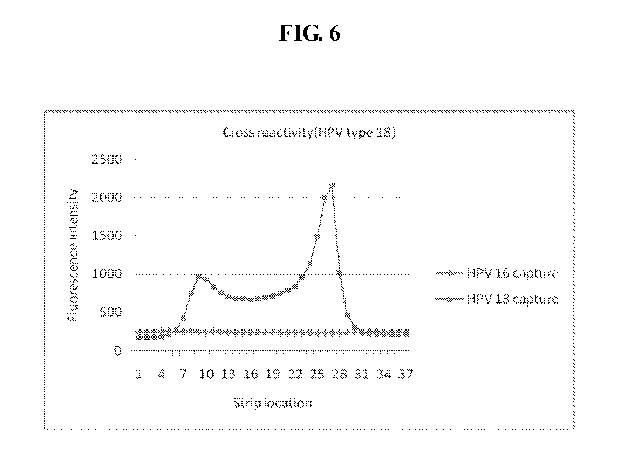

FIG 6 is the results of an assay to determine the cross reactivity among nucleic acid sequences detecting different subtypes of HPV HPV 18 in a sample was detected using a system in accordance with an embodiment of the present disclosure where the DNAs specific for HPV 16 and 18 were used as the capture molecules.

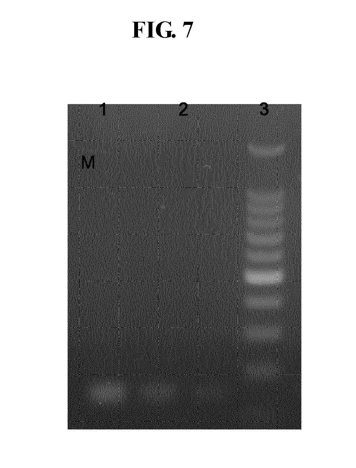

FIG 7 is the electrophoresis result run on 2% agarose gel of PCR amplified products using the primers specific for HPV 16 and 18, respectively, and labeled with biotin and using the template identified with HPV positive. Different numbers of cycles were used in PCR. M: Size marker, 100bp ladder; 1: 20 cycles of PCR; 2: 10 cycles of PCR; and 3 : 5 cycles of PCR.

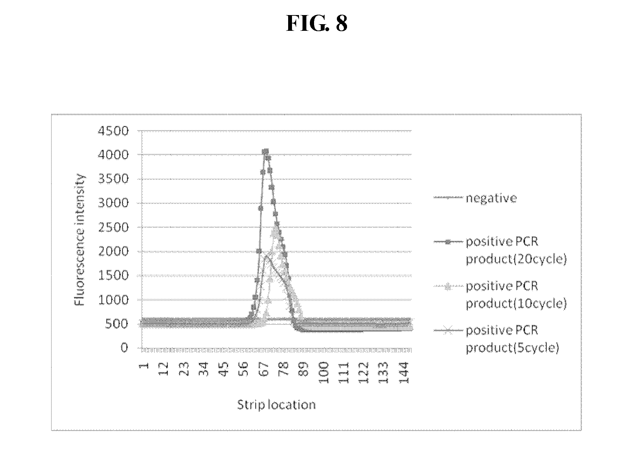

FIG 8 is the analysis result of amplified products of FIG 7 using a system in accordance with an embodiment of the present disclosure. Blue line: negative control; Red line: 20 cycles of PCR; Yellow Green line: 10 cycles of PCR; and Purple line: 5 cycles of PCR.

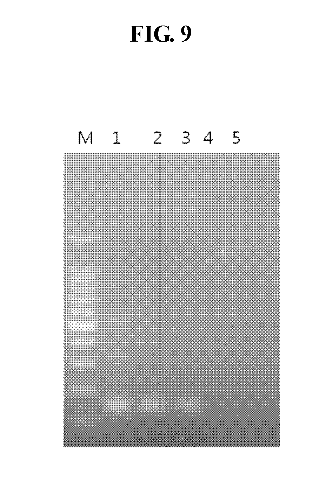

FIG 9 is the electrophoresis result run on 2% agarose gel of PCR amplified products performed under the same condition as in FIG 7 except the different sample was used as a template. M: Size marker, 100bp ladder; 1: 30 cycles of PCR; 2: 20 cycles of PCR; 3: 10 cycles of PCR; 4: 5 cycles of PCR; and 5: 3 cycles of PCR.

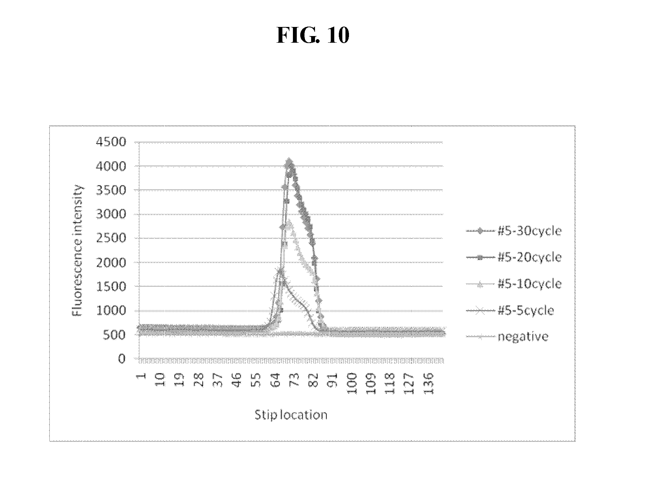

FIG 10 is the analysis results of amplified products of FIG 9 using a system in accordance with an embodiment of the present disclosure. Light Blue line: negative control; Blue line: 30 cycles of PCR; Red line: 20 cycles of PCR; Yellow Green line: 10 cycles of PCR; and Purple line: 5 cycles of PCR.

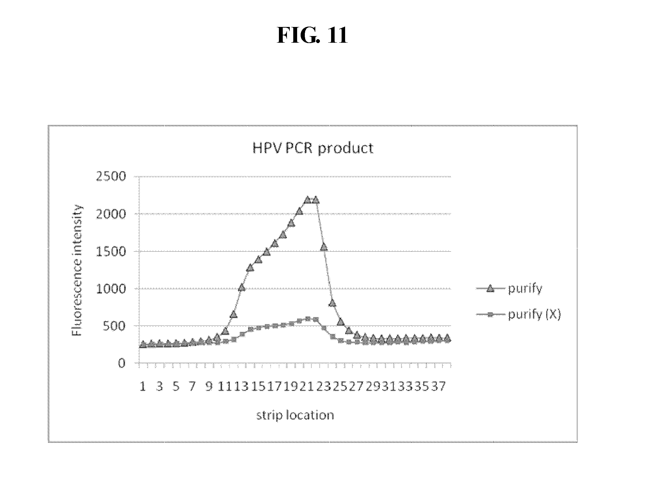

FIG 11 is the analysis results of PCR products using a system in accordance with an embodiment of the present disclosure with or without purification of the PCR products, indicated by blue and red, respectively. The products were amplified with HPV specific primers labeled with biotin and HPV positive sample was used as template.

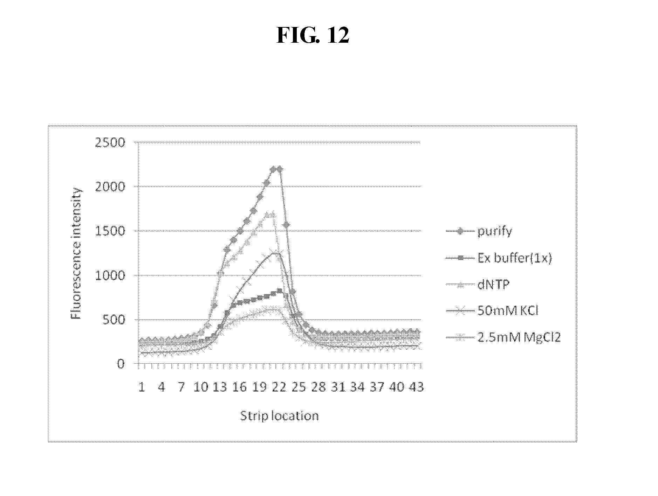

FIG 12 is the analysis results of PCR products using a system in accordance with an embodiment of the present disclosure in the presence of various materials as indicated. The products were amplified with HPV specific primers labeled with biotin and HPV positive sample was used as template. Added Components are: Blue: purification; Red: Taq Buffer; Yellow Green: dNTPs; Purple: 50mM KCl; and Light Blue: 2.5mM MgCl₂.

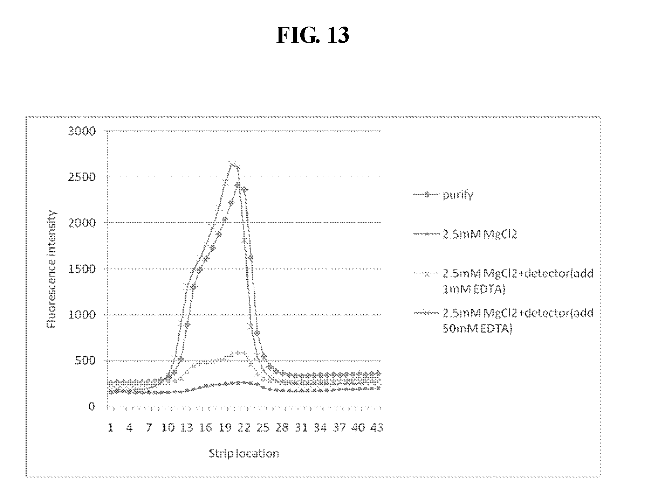

FIG 13 is the analysis results of PCR products using a system in accordance with an embodiment of the present disclosure in the presence of various concentrations of EDTA as indicated. The products were amplified with HPV specific primers labeled with biotin and HPV positive sample was used as template. Blue: purification; Red: 2.5mM MgCl₂; Yellow Green: 2.5mM MgCl₂+detector (1mM EDTA); and Purple: 2.5mM MgCl₂+detector (50mM EDTA).

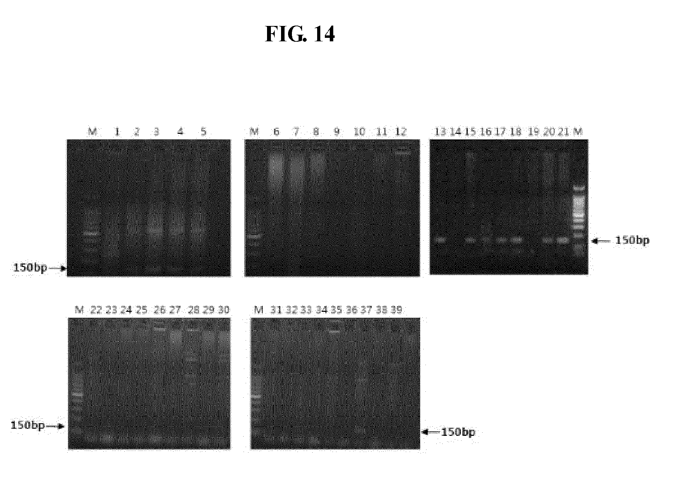

FIG 14 is the electrophoresis result run on 2% agarose gel of PCR amplified products performed using HPV specific primers labeled with biotin and various HPV positive samples as a template.
FIG 15A and 15B are the analysis results of PCR products of FIG 14 using a system in accordance with an embodiment of the present disclosure. Number on the right side of each panel indicates samples ID from different origin.

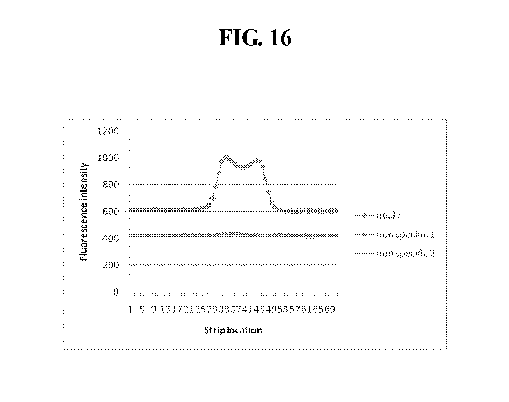

FIG 16 is the analysis results of PCR products amplified using HPV specific primers and DNA extracted from the non-specific bands of FIG 14 as template and analyzed using a system in accordance with an embodiment of the present disclosure. 37; Red: negative control.

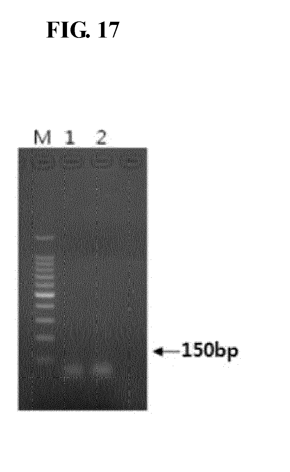

FIG 17 is the electrophoresis result run on 2% agarose gel of PCR products amplified with HPV specific primers directly from the biopsy sample without prior DNA extraction.

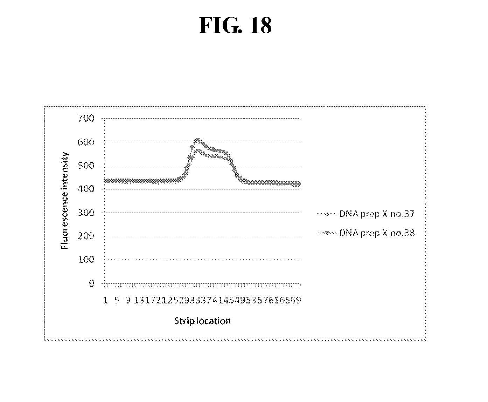

FIG 18 is the analysis results of PCR products of FIG 17 using a system in accordance with an embodiment of the present disclosure.

Like reference characters in the respective drawings indicate corresponding parts.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented here. It will be readily understood that the aspects of the present disclosure, as generally described herein, and illustrated in the Figures, can be arranged, substituted, combined, and designed in a wide variety of different configurations, all of which are explicitly contemplated and make part of this disclosure.

In one aspect, the present disclosure relates to a lateral flow strip for qualitative and/or quantitative assay of analyte, i.e., nucleic acids in a sample in a sequence specific manner.

In accordance with one aspect of the present disclosure, there is provided a strip comprising a sample pad providing a site of sample application; an absorption pad; a chromatographic medium located therebetween in a way to allow capillary flow communication with each other (the sample pad, chromatographic medium and the absorption pad) to occur, the chromatographic medium containing at least one kind of capture molecules which are capable of interacting sequence-specifically to a particular nucleic acid sequence in a sample which is applied to the sample pad; and a solid support backing the chromatographic medium, the sample pad and the absorption pad which are located on the solid support in the same plane so as to permit capillary flow communication with each other. In one embodiment, the solid support has two ends with the sample pad and the absorption pad, each being located at one end of the solid support, respectively. In other embodiment, the at least one capture molecules are immobilized at a particular location on the chromatographic medium.

The term "capillary flow communication" as used herein means that there is contact between a sample pad, chromatographic medium and an absorption pad such that a liquid sample applied in the sample pad moves through the chromatographic medium to the absorption pad by capillary action. In one embodiment, the chromatic graphic medium having a first and a second end is located between the sample pad and the absorption pad with one end being in contact with the sample pad and the other end in contact with the absorption pad wherein the ends may be overlapped with one end of the sample pad or one end of the absorption pad or may be touching or adjacent to each other. It is preferred that a sample pad, chromatographic medium and an absorption pad have the identical width, particularly where the ends are overlapped.

The term "sample" as used herein refers to a compound or a composition containing analytes to be assayed. In one preferred embodiment, the sample is a liquid or an aqueous solution such that the sample applied in the sample pad moves through the chromatographic medium to the absorption pad by capillary action.

The term "analyte" or "target analyte" or "target material" as used herein refers to a compound being analyzed in a sample and is also called "target material" and includes nucleic acids. The term "nucleic acids" as used herein refers to any DNA or RNA molecules which are derived from biological materials or chemically synthesized or amplified by known method such as Polymerase Chain Reaction, and includes but not limited to such as genomic DNA (deoxyribose nucleic acid), cDNA, RNA (Ribose Nucleic Acids). Further the nucleic acids may be double stranded or single stranded and can be extracted from the biological materials such as cells and tissues or be synthesized in accordance with the methods known in the art. In one embodiment, the nucleic acids of the present disclosure is DNA, particularly synthesized DNA by PCR, and is used as single strand and may be labeled directly or indirectly with labeling moiety for detection such as fluorescent dyes or biotin which may be incorporated into the nucleic acids during the synthesis or after the synthesis. In case where a labeling dye such as biotin is used, it is further used with streptavidin or avidin associated with particulates for detection. The particulates which may be used in the present disclosure include but are not limited to for example polystyrene microsphere, latex particulates, nanogold particulates, colloidal gold particulates, metal particulates, magnetic particulates, fluorescent particulates, and semiconducting nanocrystal particulates. The dye on the nucleic acids can be detected or be readable with an appropriate device known in the art once the labeled nucleic acids are captured to the capture molecules immobilized onto the chromatographic medium by sequence specific hybridization. The PCR amplification of the analyte, when it is required, may be performed directly on a biological specimen as template or on a DNA or RNA extracted from the specimen as template. Further the PCR products may be used analysis with or without purification after the amplification for the next step of the assay in accordance with the present disclosure.

The term "target sequence" as used herein refers to a sequence being amplified to be used as targeting material to be assayed. The targeting sequence may or may not comprise primer binding sequences. The targeting sequences may be selected in accordance with the purpose of the assay to be performed. For example, if the assay is to differentiate various types of viruses of interest, the targeting sequence should cover the area of the virus genome which is specific to that type of virus. To increase the specificity, more than one targeting sequences may be employed and for example may include sequence specific to a toxin gene or to a pathogen, which may be selected from the information known in the art. In one preferred embodiment, an illustrative example of the targeting sequence or the targeting material is derived from Human papilloma Virus (HPV), Hepatitis C Virus (HCV), HIV (Human Immunodeficiency Virus) to differentiate its subtypes. In other preferred embodiment, the targeting sequence is derived from HPV to differentiate its subtypes including high-risk and low-risk types. The high-risk HPV types include subtypes, but are not limited to, HPV subtypes 16, 18, 26, 31, 33, 35, 39, 45, 51, 52, 53, 56, 58, 59, 66, 68, 70, 73, and 82. The low-risk HPV types include subtypes, but are not limited to, HPV subtypes 6, 11, 40, 42, 43, 44, 54, 61, 72, 81 and CP108. In one preferred embodiment, the HPV subtypes include 6, 11, 16, 18, 31, 45 and 51, particularly subtypes 16 and 18.

The fluorescent dyes (molecules) which may be used in accordance with the present disclosure have about 20nm difference in wavelength between the excitation and emission wavelength. The illustrative examples may include, but are not limited to quantum dot, lanthanide chelate (e.g., Sm (samarium), Eu (europium), Tb (Terbium)) and fluorescence (e.g., FITC, Rhodamine Green, Thiadicarbocyanine, Cy2, Cy3, Cy5, Cy5.5, Alexa 488, Alexa 546, Alexa 594 and Alexa 647). In one preferred embodiment the analyte to be assayed in accordance with the present disclosure is labeled with a biotin. In other embodiment the analyte is labeled with Cy3 or Cy5. In general, the intensity of the fluorescent is proportional to that of the excitation light. The term "epifluorescence" as used herein refers to the fluorescence emitted from a conjugate of labeled targeting material or analyte with a capture molecule, and/or a reference conjugate of labeled reference material with a reference capture molecule, which can be detected from an area on the chromatographic medium where the capture molecules are immobilized.

The term "capture or capture molecule or capture oligonucleotide" as used herein refers to a material which can sequence-specifically hybridize to the analyte in a sample and includes but is not limited to nucleic acids. The capture molecule of the present disclosure is immobilized onto a particular area on the chromatographic medium and captures the analyte of interest by sequence specific hybridization which is being transferred by capillary action through the strip. The capture molecule of the present disclosure may be immobilized by a covalent or non-covalent bond to the chromatographic medium. In one preferred embodiment, the capture molecules are attached or immobilized via non-covalent bond. The methods to attach or immobilize the nucleic acids such as DNA to a porous membrane for example to a nitro cellulose membrane are known in the art and can be used for the present disclosure. An illustrative method to immobilize which can be used in the present disclosure is for example to adsorb nucleic acids onto a membrane surface followed by heat treatment at 80°C. Other illustrative method includes applying nucleic acids onto a membrane followed by air-dry and then by UV irradiation, or the capture oligonucleotides are vacuum treated in the presence of mixed with same amount of sodium chloride and sodium citrate, or UV treated. Also the capture molecule may be immobilized to a charged nylon membrane via covalent bond. In one preferred embodiment the capture molecule of the present disclosure is oligonucleotide and single stranded, which has a sequence complementary in part or in whole to that of the target material or the target analyte. In general, the capture sequence may be selected from the area of a target sequence where secondary structures due to an internal hydrogen bond are less favored. The capture molecules are applied onto a chromatographic medium such as a nitrocellulose membrane in a line, for example, with a width of about 1mm or less. The skilled person in the art may choose an appropriate line width for the application depending on the types of chromatographic medium used in the assay. The concentration of the capture molecule to be applied to the chromatographic medium of the present disclosure ranges from about 10 to 150 pM, but is not limited thereto. In one embodiment, the concentration of a capture molecule is 100 pM. At least one capture molecules, which may include but is not limited to, DNA of interest, negative and/or positive control, are immobilized onto a particular known location of a chromatographic medium on a strip of the present disclosure. The term "particular known location" means the location with a known/particular distance from the site of sample application in the direction of capillary movement (herein after referred to as "analyte measurement area") and may be included more than one depending on the number of capture molecule used. For example, the capture molecules may be present in multiple locations for example forming a line on a chromatographic medium beginning with the first one at about 2 to 5 mm from the sample application site with a space between the different capture molecules of about 1mm or more. Multiple capture molecules may be immobilized to one chromatographic medium, and the skilled person in the art would choose optimal number of capture molecules in consideration of the type of chromatographic medium used, molecular size and/or characteristics of the analytes and/or the capture molecules to be assayed. In one embodiment, the strip of the present disclosure may include same or different capture molecules in one, two, three, four, five, six, seven, eight, nine, ten, eleven, twelve, and more locations on a chromatographic medium. In cases where multiple capture molecule lines are present, the space between the lines may be various. In one embodiment, the space between the capture lines are about at least 1 mm, but is not limited thereto.

The capture molecule of nucleic acid may have a length of about 10 to 200 bases, but is not limited thereto. In one embodiment the length may include, but are not limited to, about 20 bases, 30 bases, 40 bases, 50 bases, 60 bases, 70 bases, 80 bases, 90 bases, 100 bases, 110 bases, 120 bases, 130 bases. The skilled person in art would be able to choose an appropriate sequences and/or length of the capture molecule considering the characteristics of an analyte to be assayed. Typical length is at least 20 bases or any length with a melting temperature of about 50 to 70°C. In one embodiment, the capture molecule may include space sequence consisting of 9 to 20 T residues. In other embodiment, the capture molecules may include modified nucleic acids such as PNA (peptide nucleic acid) or LNA (Locked Nucleic Acids) to alter or improve hybridization characteristics, which is particularly useful for short oligonucleotides or when the regulation of melting temperature is needed. The capture molecules are designed to hybridize to the analyte of interest preferably within 0, 1 or 2 bases difference. This is because that the base stacking is important in a stable hybridization. The sequence of a capture molecule is selected in consideration of the analyte of interest. Therefore more than one capture molecules with various sequences in accordance with the number of different types of analyte to be assayed may be used. In one embodiment, the capture molecule is derived from HPV sequences including high-risk and low-risk sub types. Illustrative examples of the high-risk subtypes in accordance with the present disclosure include but are not limited to subtypes 16, 18, 26, 31, 33, 35, 39, 45, 51, 52, 53, 56, 58, 59, 66, 68, 70, 73, 82. Illustrative examples of the low-risk subtypes in accordance with the present disclosure include but are not limited to subtypes 6, 11, 40, 42, 43, 44, 54, 61, 72, 81, CP108. In one embodiment, the HPV subtypes include 6, 11, 16, 18, 31, 45 and 51. In other embodiment, the HPV subtypes include 16 and 18.

Further the strip of the present disclosure may be enclosed in a housing (case) for hygienic reason for example to avoid contamination. The housing which may be used for a strip of the present disclosure may cover the entire strip except areas of sample application and epifluorescence measurement. In one embodiment, an illustrative example of a housing as depicted in Fig 2 (110) may be used.

In other aspect the present disclosure provides a nucleic acid detection system comprising any one of the strip in accordance with the present disclosure and epifluorescence detection device to detect the signal emitted from the location where capture molecules are immobilized and the analytes of interest are hybridized thereto.

Epifluorescence detection device known in the art may be used for the present system. Generally the laser induced epifluorescence detection device which can be used with the present system comprises a laser, an excitation filter, an elliptical reflecting mirror or spherical mirror, epifluorescence control means, a collimator, a fluorescent filter and an optical detector. An illustrative example of the detection device which may be included in the present system is depicted in Fig. 2. The principle of detecting and collecting epifluorescence is explained referring to Fig. 2. Incident light from the laser that passes through the excitation filer is focused on the first point of the elliptical reflecting mirror where the sample/analyte is located and the light focused on the second point of the elliptical reflecting mirror is converted into parallel light which then passes through fluorescent filter to condensing lens and to optical detector. The epifluorescence detected by the optical detector then is transferred to CPU via analog digital converter to determine the presence or absence and/or the amount of the analyte present in the sample in relative comparison to a standard fluorescence. In one embodiment, the detection devices which may be used for the present system include but are not limited to, *i-CHROMA®* from Boditech Med, Inc. (Korea) or a device from Biosite (USA).

In other aspect the present disclosure provides methods for detecting nucleic acids in a sample using the strip in accordance with the present disclosure as described herein. The method comprises steps of : applying a liquid sample containing a nucleic acid sequence(s) of interest labeled with a fluorescent or a biotin to a sample pad of the strip of the present disclosure; and allowing the liquid sample to flow through a chromatographic medium to an absorption pad of the strip, wherein the capture on a particular location on the chromatographic medium of the strip reacts sequence specifically with the nucleic acid sequences of interest in the sample to form a hybrid ; and detecting a signal emitted from the labels on the hybrid using a epifluorescence detection device, where the presence of the signal indicates the presence of the nucleic acid of interest or the signal is used to quantify the amount of the nucleic acid of interest in the sample. In one embodiment, the capture molecules have a nucleic acid sequence derived from Human Papilloma virus (HPV).

The present method is particularly useful for sequence specific detection of DNA or RNA in a sample. Typically whole DNA or RNA is extracted from cells and tissues and then the DNA of interest is amplified using PCR. The amplified DNA is then applied to a strip of the present disclosure containing a capture molecule on a chromatographic medium, whose sequence is complementary in whole or in part to the amplified sequence. After the reaction the signal is then detected using an epifluorescence detection device. The method in accordance with the present method is very sensitive and capable of detecting PCR products whose amplification cycle is 5 or less. It is also possible to using a biological sample directly for the PCR as a template without prior purification of nucleic acids from the sample. In case where prior purification of nucleic acids from a sample is required, one in the art would be able to choose from various methods known in the art and also from commercial products and kits to extract and purify DNA and RNA. Typically the amplified products, i.e., the target analyte, are used as single strand. The target sequences comprise a primer binding site. The target sequence to be assayed may be various and differ depending on the purpose of the assay. For example, when the assay is to differentiate various subtypes of a virus within one species, the target sequence should be selected to cover the area of a virus genome which contains sequences unique to each subtypes of the virus. More than one target sequence may be employed to improve the specificity and/or sensitivity. There are provided various information in the art which can be used for example, for selecting pathogen or toxin specific sequence.

The target analytes may be labeled with a labeling dye directly or indirectly. The labeling dye may be either added into a liquid sample just before application onto a strip or be incorporated into the amplifying DNA during the amplification process. For example, the target sequences may be amplified or synthesized to include in it the fluorescent molecules or detection moiety such as biotin. In the latter case, the biotin-labeled sequences are then reacted with a particulate associated with streptavidins to be used as a detection material. Various particulates known in the art which may be used for this purpose include but are not limited to for example polystyrene microsphere, latex particulates, nanogold particulates, colloidal gold particulates, metal particulates, magnetic particulates, fluorescent particulates, and semiconducting nanocrystal. The nucleic acids labeled with a dye now can be detected or be readable with an appropriate device known in the art once the labeled nucleic acids are captured onto the captured molecules immobilized onto the chromatographic medium by sequence specific hybridization. The PCR amplification of a target sequence, when it is required, may be performed with a primer labeled with a detection material, which methods are known in the art. The nuclei acid molecules whether it is amplified or is used without amplification are transferred by capillary action from the application site on a sample pad and flow through a chromatographic medium where the analytes hybridize sequence-specifically to a capture molecule deposited and immobilized at a particular site on the chromatographic medium to form an analyte-capture complex. Thereafter the signal from the complex (hybrid) is detected with an epifluorescence detection device, and the positive signal from the complex indicates the presence of nucleic acids of interest in a sample.

The strip, system and method in accordance with the present disclosure can be used for example to differentiate various subtypes of a virus or to identify the presence of a particular pathogen. In one embodiment, the strip, system and method in accordance with the present disclosure can be used to differentiate and identify various subtypes of HPV virus. HPV is a virus having 7900 bp double strand DNA as its genome and is found in 95% of women with cervical cancer and considered to be the main cause of a cervical cancer. To date about 120 different subtypes are found, among which about more than 40 subtypes are found to be associated with cervical cancer which are further classified into high and low risk types. The high risk type includes HPV subtypes 16, 18, 26, 31, 33, 35, 39, 45, 51, 52, 53, 56, 58, 59, 66, 68, 70, 73, and 82, and the low risk type includes HPV subtypes 6, 11, 40, 42, 43, 44, 54, 61, 72, 81, and CP108, which can be detected using the present strip, method and/or system. Among the high risk types, subtypes 16 and 18 represent about 70-80%, the remaining 20-30% are represented by subtypes 31, 33, 35, 52, and 58. In a minority of cases, HPV can also lead to cancer of the penis. (Gissmann L, Boshart M, Durst M, Ikenberg H, Wagner D, zur Hausen H. Presence of human papillomavirus in genital tumors. J Invest Dermatol 1984;83 (1 suppl): 26S-8S; de Villiers EM. Heterogeneity of the human papillomavirus group. J Virol 1989;63: 4898-903; Lorincz AT, Reid R, Jenson AB, Greenberg MD, Lancaster W, Kurman RJ. Human papillomavirus infection of the cervix: relative risk association of 15 common anogental types. Obstet Gynecol 1992;79:328-37.)

The common types of cervical cancer are Squamous cell carcinoma (SSC), which represent about 90-95% of all cases, and adenocarcinoma. The precancerous lesion of SSC is identified as dyskaryosis where the nuclei of cells have abnormal shape or cervical intraepithelial neoplasia (CIN) using various methods such as cytodiagnosis, colposcopic examination or biopsies. CIN is further classified into first, second and third stages depending on the progression of the CIN (McIndoe WA et al.Obstetrics and Gynaecology, October, 1984;64(4):451-458). As described in Examples of the present disclosure, the strip, method and system of the present disclosure can differentiate different subtypes of HPV present in a various cervical cancer lesions. In a preferred embodiment, the strip, method and system of the present disclosure can be used to identify and differentiate HPV subtypes 16 and 18.

It is imperative to determine the HPV infection not only for the prevention of the cervical cancer but for the prognosis after/during cancer treatment because of a high rate of cervical cancer in women and that HPV is considered to be a main cause. Prior methods to diagnose cervical cancer includes cytodiagnosis, colposcopic examination, or biopsies, immunological test to detect HPV proteins, and HPV DNA test, among which HPV DNA test is on a constant rise due to its sensitivity, specificity and accuracy. (Duggan MA, Benoit JL, Mcgregor SE, Nation JG Inoue M, Stuart GC. The human papillomavirus status of 114 endocervical adenocarcinoma cases by dot-blot hybridization. Hum Pathol 1993;189:12-9; Jane C. Sterling and Stephen K. Tying. Human Papillomavirus: Clinical and scientific advances, ARNOLD, 2001; Schneider A, Zahm DM, Kirchmayr R, Schneider VL. Screening for cervical intraepithelial neoplasia grade 2/3: validity of cytologic study, cervicography, and human papillomavirus detection. Am J Obstet Gynecol 1996;174:1534-41; Cuzick J, Beverley E, Ho L, Terry G, Sapper H, Mielzynska I, et al. HPV testing in primary screening of older women. Br J Cancer 1999;81:554-8.) Particularly, the cytodiagnosis has problems of high rate of false negative, low sensitivity and the inaccuracy of diagnosis. The following Table 1 compares the commonly used DNA HPV tests.

**[Table 1]**

| | **HPV typing kit** | **PCR Test** | **Hybrid Capture** | **HPV DNA Chip** |
|---|---|---|---|---|
| **Sensitivity** | High | High | High | High |
| | | | | |
| **Specificity** | High | High | High | High |
| | | | | |
| **Test Time** | 3∼4 hour | 6 hour | 3∼4 hour | 5 hour |
| | | | | |
| **Number of subtypes identified** | 2 subtypes (16, 18) | 2 subtypes (16, 18) | 17 subtypes of high risk, 12 subtypes of low risk | 22 subtypes of high risk, 15 subtypes of low risk |
| | | | | |
| **Detection method** | Fluorescent/color detection | ETBR Staining | Luminometer | Chip Scanner |

The HPV typing kits of Table 1 is a typical PCR based kit including HPV typing kit from GENOMED Inc., where the sequences specific to each subtypes are amplified and run on a agarose gel which then visualized with ETBR staining. The Hybrid Capture is a test based on RNA-DNA hybrid formation which is then detected with antibody specific to the hybrid and includes a product from Diagene Inc.(U.S.A.). HPV DNA Chip includes a product from Biomedlab (Korea).

The present method has advantages compared to the prior methods in terms of specificity and sensitivity in addition to short reaction-to-result time. Specifically, the present method has a short sample preparation time because the test can be done on a sample with only 5 cycles of PCR for example to detect particular subtypes of HPV, and in addition, PCR may even be performed on a biopsy without DNA purification. In the next step for detection, the amplified products can be used directly without purification on the strip for chromatography separation, which requires only about 10 minutes to see the results. These all add up to a reduced reaction-to-result time compared to the prior tests. Moreover, analysis time may be further reduced by employing more than one capture molecules. For example as indicated in the Examples, total of about 30 min to 90 min analysis time is required: DNA extraction from biopsy if required: about 1 hour; amplification by PCR of 5 cycles: about 20 min; and chromatographic separation and detection: 12min. This can reduced the total analysis time to 1/20.

Now the strip, and the system comprising the laser induced epifluorescence detection device and the strip of the present disclosure is explained in detail by referring to the appended figures.

### Solid support or backing cards

The solid support (101) supports all the other components, a sample pad, a chromatographic medium and a absorption pad of the strip, or when it is not used, the chromatographic medium (104) per se can function as a support. The support is typically made of water insoluble, nonporous and rigid material and has a dimension equal to or more than the dimension of all the other components combined on the support. The support may be prepared from various natural and synthetic organic and inorganic materials, as long as that the support does not prevent or interfere with the capillary flow through the strip and interfere with the interaction between the target analyte and the capture molecule in addition to no non-specific binding to the analytes. Illustrative examples of the materials which may be used for the present support include, but are not limited to, polyethylene, polyester, polypropylene, poly(4-methylbutene), polystyrene, polymethacrylate, poly(ethylene 60 terephthalate), nylon, poly(vinyl butyrate), glass, ceramic, metal and the like. The other components of the strips can be attached to the support by various means including such as adhesives. Proper selection of adhesives may improve the performance of the strip and lengthen the shelf life of the strip. An adhesive which may be used in accordance with the present disclosure includes but is not limited to pressure-sensitive adhesive (PSA). Typically the attachment between the support and the other components of the strip is accomplished as the adhesive penetrates into the pores of the other components, thereby binding them together on the support. This ability of an adhesive to flow under normal conditions is referred to as "cold flow". Since no heat is applied when applying PSA on to the strip components, cold flow of a certain level is indispensable for binding between the strip components. If the level of cold flow is too low, the initial binding force become low, causing insufficient binding between the strip components. In contrast, if the level of cold flow is too high, the adhesive migrates into the other components of the strip and may cause clogging of the pores, formation of hydrophobic spots or redampening of the strip. Such problems associated with the cold flow of the adhesive can be solved by using a direct-casting of membranes. For example, in the direct-casting, a supporting plastic sheet prevents the adhesive from entering into the pores of the membrane and thus vertical migration of the adhesive is prevented during storage.

### Sample Pads

The sample pad (102) is located at one end of the strip and in capillary flow communication with the chromatographic medium (104). Basically the sample pad serves to receive the fluid sample containing a target analyte to start a capillary flow of the analyte and needs to have a minimum binding activity toward nucleic acids. Also it may filter insoluble particles in the sample. In consideration of these functions, preferred sample pads which may be used for the present disclosure include but are not limited to cellulose filter paper or glass fiber filter paper capable of providing the filtering function. For example Millipore cellulose fiber material (Cat# CFSP223000) may be used as the sample pad. Preferably, the sample pad is pretreated to prevent the analyte in the sample from being non-specifically adsorbed thereto, to allow the components of the sample to be transferred readily through the chromatography medium, and to maintain the sensitivity of the reaction. The pretreatment of the sample pad is generally performed by treating the pad with an inactive protein or surfactant. For instance, the pretreatment is carried out by immersing the pad material in a solution of 0.1 to 10% bovine serum albumin (BSA)-containing 0.1 M Tris buffer solution (pH 6-9), a solution of 0.1 % to 10% skim milk powder in 0.1 M Tris buffer solution (pH 6-9) and/or 0.1 to 10% casein solution. The pretreatment with a surfactant is carried out by immersing the pad in for example, 0.01% to 1% solution of Triton X-100 or Tween® 20, non-ionic surfactant, followed by vacuum drying at high temperature. Preferably, the sample pad may be treated with an inactive protein and then a surfactant. However, these specific steps, conditions and reagents used pretreatment steps may be various and determined in accordance with characteristics of analytes and samples used in the assay.

### Chromatography Media

The chromatography medium (104) is in capillary flow communication with the sample pad (102) and absorption pad (103) at its each end. The chromatography medium (104) may be supported by the solid support (101), in which case it can attach to the support as described above, or it may serve as a solid support per se. Materials that can be used as the chromatographic medium includes any materials which can allow the liquid in a fluid sample and the analyte, particularly nucleic acids therein to be transferred through the material via capillary action to have interaction with the capture molecule immobilized thereon, and which can be modified to covalently or non-covalently bind to the capture molecules. The capture molecules may be immobilized on the chromatography medium for example via chemical bonding. The chemical bonding is carried out according to a known method (LABORATORY TECHNIQUES IN BIOCHEMISTRY AND MOLECULAR BIOLOGY, Volume 15, Edited by R. H. BURDON and P. H. Van KNIPPENBERG ELSEVIER AMSTERDAM: NEW YORK, OXFORD (1985) P. 318-322). Typically, the chromatography medium refers to a porous material which can allow capillary flow by aqueous solution. The chromatographic medium which may be used for the present disclosure include but is not limited to for example, cellulose, nitrocellulose, polyethersulfon, polyvinylidine fluoride, nylon, charged nylon, ceramics and polytetrafluoroethylene. In one embodiment, nitrocellulose is used for the chromatographic medium, the pore diameter of which is at least 0.1 µm, particularly at least about 1.0µm, more particularly about 0.2µm to about 20µm, most particularly about 0.2µm to about 12µm. In cases where particulate materials are used as labeling means, the pore size should be at least 10 times the size of the particulate used. The chromatography medium may be multifunctional or be modified to be multifunctional to covalently bind to the capture molecules. A preferred chromatography medium which can be used for the present disclosure includes Prima 60, 85, AE98, AE99 and AE100 from Schleicher & Schuell Bioscience Inc.; HiFlow Plus HF09004, HF13504,HF090, HF120, HF135, HF180 and HF240 from Millipore; and CN90, CN140 from Sartorius AG

### Absorption pads

The absorption pad (103) is located on one end of the solid support opposite to the sample pad and is in capillary flow communication with one end of the chromatographic medium. The absorption pad is to physically absorb any liquid and remove any unreacted materials, if any, which has passed through the sample pad and chromatographic medium by capillary action. The absorption pad which is located at the end of the strip controls or promotes the speed of capillary movement by which the analyte and liquid sample are transferred and also works as a reservoir to contain them. The speed of transferring material may vary depending on the size and quality of the absorption pad used. An illustrative example of the absorption pad which may be used for the present disclosure includes nitrocellulose, cellulose ester, glass (for example, borosilicate glass fiber), polyethersulfon, cotton, dehydrated polyacrylamide, silica gel, and polyethylene glycol and the like. The speed of capillary flow may be selectively controlled by suitable selection of the absorption pad.

### Laser induced epifluorescence detection device

In order to measure epifluorescence to determine the quantities of analytes, a laser-induced epifluorescence detecting device is used. Fig. 2 is an illustrative example of Laser induced epifluorescence detection system of the present disclosure, which comprises a laser (201), a lens to control laser beam (202), an exciter filter, a collector lens (203), a fluorescent filter (204), a condensing lens (205), a spatial filter (206), light detector (207), Analog Digital Converter (ADC) and Central Processing Unit (CPU). Illustrative examples of the detection device which may be used for the present disclosure include ones descried in Korean Patent No. 063776 by the present applicant, GSI scanner ( the GSI group Inc., USA) or Axon(Axon Instruments Inc., USA). In general, parallel laser beam from the laser induced epifluorescence detection device which has passed through the excitation filer is focused on the sample and then the fluorescent dye on the capture -analyte complex emits lights which are in turn directed to the center of a spatial filter via a collector lens, a fluorescent filter and condensing lens and reach the light detector, where the signal is converted to a readable format by ADC and CPU.

In other words, the laser induced epifluorescence detection device is used to detect the signal from the capture -analyte (nucleic acid molecules) complex which are formed by sequence specific hybridization between them and the fluorescent signal from the dye on the target analyte is collected and converted by the device to quantify the analyte in a sample. During that process, after the chromatography is completed, the strip is moved to a light source and is scanned for the collection and detection of the fluorescent signal from the strip. Particularly, the signal from the strip may be scanned more than once to increase the sensitivity.

Now, the lateral flow assay strip according to the present invention and the method for detection and/or quantification of the analyte using the strip will be more concretely described while referring to Figs 1 and 2. From this description, the features and advantages of the present disclosure will become more apparent.

Fig. 1 depicts an illustrative example of a strip for lateral flow assay (100) to detect nucleic acids in a sample. The later flow assay begins by applying the sample to a sample application site (109) on the sample pad (102). The aqueous sample now starts to flow by capillary action through the strip. The speed of the movement may be affected by the types, quality and size of the absorption pad used. When the nucleic acids are labeled with biotin, beads coated with streptavidin are mixed into the sample before the application. A area (105) on the strip where the known amount of capture molecules is immobilized is located at a known distance from the sample application site. The capture molecule contains a sequence which can bind to the nucleic acids in the sample by sequence specific hybridization and due to its immobilization to the chromatographic medium, is not transferred through the strip. Thus the signal from the capture-analyte complex can be detected by a detection device at the region (105, 108) of the chromatographic medium of the strip where the capture molecules are immobilized.

In order to determine the amount of a target molecule present in a sample, a standard curve of the analyte of interest is constructed using an analyte of kwon concentration. This curve is then used as a reference standard for extrapolating quantitative information for nucleic acid targets of unknown concentrations.

The present invention is further explained in more detail with reference to the following examples. These examples, however, should not be interpreted as limiting the scope of the present invention in any manner.

The present disclosure except where indicated otherwise is readily practiced using the knowledge, information and techniques which are within a level of a person skilled in the art. The following books and publications may be referred for general information on molecular biology and biochemistry : Molecular Cloning: A Laboratory Manual, 3rd Ed. (Sambrook et al., Harbor Laboratory Press 2001); Short Protocols in Molecular Biology, 4th Ed. (Ausubel et al. eds., John Wiley &Sons 1999); DNA Cloning, Volumes I and II (Glover ed., 1985); Oligonucleotide Synthesis (Gait ed., 1984); Nucleic Acid Hybridization (Hames and Higgins eds. 1984); Transcription And Translation (Hames and Higgins eds. 1984); Culture Of Animal Cells (Freshney and Alan, Liss, Inc., 1987); Gene Transfer Vectors for Mammalian Cells (Miller and Calos, eds.); Current Protocols in Molecular Biology and Short Protocols in Molecular Biology, 3rd Edition (Ausubel et al., eds.); Recombinant DNA Methodology (Wu, ed., Academic Press); Gene Transfer Vectors For Mammalian Cells (Miller and Calos, eds., 1987, Cold Spring Harbor Laboratory); Methods In Enzymology, Vols. 154 and 155 (Wu et al., eds.); Immobilized Cells And Enzymes (IRL Press, 1986); Perbal, A Practical Guide To Molecular Cloning (1984); the treatise, Methods In Enzymology (Academic Press, Inc., N.Y); Immunochemical Methods In Cell And Molecular Biology (Mayer and Walker, eds., Academic Press, London, 1987); Handbook Of Experimental Immunology, Volumes I-IV (Weir and Blackwell, eds., 1986); Protein Methods (Bollag et al., John Wiley &Sons 1996); Immunology Methods Manual (Lefkovits ed., Academic Press 1997); and Cell and Tissue Culture: Laboratory Procedures in Biotechnology (Doyle &Griffiths, John Wiley &Sons 1998). The reagents, cloning vector and various kits and dyes may be purchased from companies such as BioRad, Stratagene, Invitrogen, Sigma-Aldrich,and ClonTech and the like.

### EXAMPLE 1

### Preparations of the target analyte and the capture molecules

To prepare a strip to test HPV DNA, target analytes were prepared as follows. First, genomic DNAs were extracted from biopsy tissues which were identified as HPV subtype 16, 18 or HPV positive (biopsy samples were obtained from Yonsei University of Seoul, Korea and types were identified using DNA chip). Extracted genomic DNAs were further purified by phenol/chloroform extraction where1ml of DNA solution were mixed with 1ml of phenol/chloroform/Isoamyl alcohol (25:24:1) followed by gentle vortexing and centrifugation at 14,000 rpm for 5min at Room Temperature. After the centrifugation, the supernatant was transferred to a fresh tube and two volumes of 100% ethanol at -20°C was added thereto and mixed well. The supernatant of the mixture was then removed by centrifugation at 14,000rpm at 4°C for 15 min and the pellet was washed gently with 70% EtOH. The residual EtOH was removed by centrifugation at 14,000rpm at 4°C for 15 min, and the pellet was dried at 37°C and dissolved in 30µl of distilled water and kept at -20°C before use. Thereafter the region of HPV genome that determines its subtypes, i.e., target sequence (analyte), was amplified using the primer as listed in the table 2 below. All the antisense primers were labeled with biotin. Specifically 2µl of DNA from each biopsy sample as prepared above were mixed with 1µl of each of primer No. 1 and 2 (or No. 1 and 3). The components used in the PCR reaction was as follows: 10 x Hot prime Taq^{™}buffer 5µl, dNTP mixture 4µl, 25mM MgCl₂ 4µl, Hot prime Taq^{™}polymerase 0.7µl, D.D.W 32.3µl. The PCR reaction condition used was performed as follows: 94°C, 2 min X 1 cycle, (94°C, 30 sec, 53°C, 30sec, 72°C, 1min) X 30 cycles, 72°C, 5min X 1 cycle. The primers and the capture DNAs were synthesized from Genotech (Korea) and the Taq polymerase was obtained from Takara (Japan, Ex Taq and Ex buffer). The amplified PCR products were purified using PCR purification kit from MP Biomedicals, LLC in accordance with the manufacturer's instruction.

The primers used for the amplification were selected from L1 region ofHPV by reference to the following publications: (Jianduan Li et al., Denaturing High-Performance Liquid Chromatography for Detecting and Typing Genital Human Papillomavirus. J of Clinical Microbiology 2003;41(4):5563-5571; A J van den Brule et al., Journal of Clinical Microbiology. 1990; 28(12):2739-2743; and Ana-Maria de Roda Husman et al., Journal of General Virology. 1995:76; 1057-1062.) The capture sequences were selected by reference of the following publications: Ana-Maria de Roda Husman et al. The use of general primers GP5 and GP6 elongated at their 3' ends with adjacent highly conserved sequences improves human papillomavirus detection by PCR. J. of General Virology. 1995;76:1057-62; P.E. Gravitt et al., Improved amplification of genital human papillomaviruses. J of Clinical Microbiology. 2000; 38(1):357-61; Bernhard Kleter et al. Novel short-fragment PCR assay for highly sensitive broad-spectrum detection of anogenital human papillomaviruses. American J of Pathology. 1998;153(6):1731-9; Peter J. F. Snijders et al. The use of general primers in the polymerase chain reaction permits the detection of a broad spectrum of human papillomavirus genotypes. J of General Virology. 1990;71:173-81)

**[Table 2]**

| Primer No. | Type | Sequence | Size (bp) |
|---|---|---|---|
| 1 | HPV sense primer | 5'-TTT GTT ACT GTG GTA GAT ACT A-3' | 22 |
| 2 | HPV anti-sense primer-I | Cy5-5'-TGA AAA ATA AAC TGT AAA TCA TAT-3' | 24 |
| 3 | UPV anti-sense primer-II | biotin-5'-TGA AAA ATA AAC TGT AAA TCA TAT-3' | 24 |
| Capture molecule | HPV type 16 capture | 5'-ACG CAG TAC AAA TAT GTC ATT ATG TGC TGC CAT ATC TAC TTC AGA AAC TAC | 96 |
| | | ATA TAA AAA TAC TAA CTT TAA GGA GTA CCT ACG ACA TGG GGA GGA -3‘ | |
| Capture molecule | HPV type 18 capture | 5'-CAC TCG TAG TAC CAA TTT AAC AAT ATG TGC TTC TAC ACA GTC TCC TGT ACC TGG GCA ATA TGA TGC TAC CAA ATT TAA GCA GTA TAG CAG ACA TGT TGA A -3' | 100 |

### EXAMPLE 2

### Pretreatment of the Sample Pad

The sample pad (S&S 903) was pretreated to increase the flow of the components/analyte in the liquid sample and to maintain the sensitivity and to minimize the nonspecific binding of the target analyte and/or the capture DNA to the nitrocellulose membrane. The sample pad (2.5 x 30cm) was immersed in a Phosphate Buffered Saline (PBS, 137 mM NaCl, 2.7 mM KCl, 10 mM Sodium Phosphate dibasic, 2 mM Potassium Phosphate monobasic pH of 7.4) comprising 1% BSA, 0.05% Tween® 20 for 10 min for equilibration. Then excess solution was removed from the pad followed by vacuum dry at 50°C to prevent deformation. The pretreated sample pads were kept in dry condition until use.

### EXAMPLE 3

### Immobilization of the capture sequence to a nitrocellulose membrane

Nitrocellulose cellulose membrane (Millipore, HF180) was used as a chromatographic medium. The capture DNA as prepared in Example 1 was diluted with distilled water to 100pM and then boiled at 95°C for 5 min and further diluted 10 times with 3X SSC (Sodium Chloride/Sodium Citrate buffer)(Sigma-Aldrich, USA). The prepared capture DNA was then deposited using microdispenser from Bio-Dot (USA) at the amount of 1µl/cm to form a line of 1mm width on an area on the nitrocellulose membrane which is at 2.8 mm from the sample pad. After the deposition of the capture DNA, the nitrocellulose membrane was dried at 42°C for 1 hour followed by UV irradiation at 1200J for 5min to fix the capture DNA, which was found to be the most effective condition tested for fixing (Fig. 3). The strip prepared above were assembled onto a support with other components of the strip as described below in Example 4, which is then inserted into a disposable cassette/housing (16 x 90mm) which is compatible for use with laser induced epifluorescence detector from Boditech Med Inc. of Korea (laser-fluorescence scanner, *i-CHROMA*®). The assembled strips were then single packaged under dry condition and kept at RT before use.

### EXAMPLE 4

### Preparation of the strip

The sample pad and the Nitrocellulose membrane as prepared above in Examples 2 and 3, respectively, and the absorption pad (cellulose membrane, 3M chromatography grade) and the solid support (PJ Inc. Korea, Plastic support with adhesive) were assembled into a disposable cassette (16 x 90mm). The NC membrane prepared as in Example 3 was cut to 25 mm x 300 mm and placed at the center of the support of 89mm x 300mm in size. Then the sample pad was placed at one end of the support and the absorption pad was placed at the other end of the support so as to overlap 0.2mm with a respective end of the NC membrane at the center. The assembled strip then was cut to 4.11 mm x 64.8 mm and inserted into a disposable cassette. The assembled strips were then single packaged under dry condition and kept at RT before use.

### EXAMPLE 5

### Preparation of fluorescent bead and avidin complex

100mM MES buffer (2-(N-morpholino) ethanesulfonic acid, pH 6.0, 25mM NaCl, 2% bead solution (Alexa 647 fluorescent bead from Molecular Probes, 0.2µm in diameter, the surface is carboxilated), 0.2mg EDC(ethylene dichloride) and 0.5mg sulfo-NHS were mixed well in RT and allowed to react for about 15min at RT followed by 1min sonication. After the sonication 15 µl of 2-mercaptoehanol was added to the mixture and allowed to react for 5min to remove unreacted EDC followed by another round of 1 min sonication. After the second sonication, 100µg of Avidin(Kem & Tec Inc.) was added followed by incubation for 2 hours at RT and then 75µl of 1M glycine (pH7.2) was added followed by incubation for 30 min at RT. After that, centrifugation at 14,000rpm for 5min was done to remove the supernatant and the pellet was washed 3 times with wash buffer (50mM NaPi pH7.4, 50mM NaCl). After the last washing step, blocking buffer (50mM NaPi pH7.4, 50mM NaCl, 1% BSA, 0.1% Tween® 20) was added and allowed to react for 1 hour at 4 °C followed by stabilization of the beads by adding stabilization buffer (50mM NaPi pH7.4, 50mM NaCl, 1% BSA, 0.1% Tween® 20, 25% Trehalose, 0.1% sodium azide).

### EXAMPLE 6

### Hybridization assay of HPV PCR products using the strip

PCR product obtained using the primer labeled with Cy5 prepared as described in Example 1 was mixed with 0.4 N NaOH at 1:1 ratio to denature and allowed to react at RT for 3min. After the incubation, neutralization buffer (1M Tris-Cl, 0.3% Tween® 20, pH6.3) was added at 1:100 ratio, 100 µl of which was then applied to the sample pad of the strip with the corresponding capture molecules immobilized thereon as prepared in Examples 3 and 4 and was left for 12 min for lateral flow movement to occur. After 12 min, the strip was then scanned with fluorescence detection device, *i-CHROMA*® (Boditech Med Inc. Korea).

With regard to biotin labeled primers, PCR product obtained using the primer labeled with biotin prepared as described in Example 1 was mixed with 0.4 N NaOH at 1:1 ratio and allowed to react at RT for 3min. After the incubation, 50µl of neutralization buffer (1M Tris-Cl, 0.3% Tween® 20, pH6.3) and 0.1 % fluorescent beads as prepared in Example 5 at 1:10 ratio were added and mixed well. 100 µl of the mixture was then applied to the sample pad of the strip with the corresponding capture molecules immobilized thereon as prepared in Examples 3 and 4 and was left for 12min for lateral flow movement to occur. After 12 min, the strip was then scanned with fluorescence detection device, *i-CHROMA* ®(Boditech Med Inc. Korea). The buffer used for chromatography separation was PBS containing 1% gelatin, 0.3% Tween® 20, 0.1% Thimerosal. The strip location is the relative position automatically determined by the device.

### EXAMPLE 7

### Specificity of the HPV detection and Cross reactivity test

PCR was done using genomic DNA from the biopsy sample determined to be HPV 16 or 18 as descried in Example 1 as a template and Cy5 labeled primers to obtain a 150bp product. The amplified products were electrophoresed on a 2% agarose gel (Fig. 4). Also the products were treated as described in Example 6 and applied to the strip prepared as described in Example 4 and was scanned for 12 min using *i-CHROMA*® (Boditech Med Inc.) The strip has the capture molecule specific to HPV 16 or 18 as described in Example 1 immobilized thereon. The results are shown in Figs. 5 and 6. As it can be seen from the figures, the signal for HPV 16 target sequence (analyte) is only detectable on the site where the capture molecule specific to HPV 16 are present, the signal for HPV 18 target sequence (analyte) is only detectable on the site where the capture molecule specific to HPV 18 are present. Thus the results indicate that there was no cross reaction between HPV 16 and 18.

### EXAMPLE 8

### Sensitivity of the HPV detection

Two independent biopsy samples identified HPV-positive was used for the experiments. PCR was performed on the genomic DNA extracted from the sample using primers (Nos. 1 and 3 of Table 2) labeled with biotin under the same condition as done in Example 1 except that 3, 5, 10, 20 and 30 cycles of PCR was performed. The amplified products were electrophoresed on a 2% agarose gel (Fig. 4). Also the products were treated as described in Example 6 and applied to the strip prepared as described in Example 4 and was scanned after 12 min using *i-CHROMA* ®(Boditech Med Inc.) for fluorescent detection. The results are shown in Figs. 7 to 10. As it can be seen from Figs. 7 and 8, when using the strip in accordance with the present disclosure, even 5 or less cycles of PCR is enough to detect the positive fluorescence from the capture-analyte hybrid, which is in contrast to the negative result obtained with electrophoresis method (2% agarose gel). The same results were obtained using other samples of independent origin (Figs. 9 and 10).

### EXAMPLE 9

### Use of the analyte amplification products without purification in the assay

In general PCR products are purified after amplification for the next step of the analysis. However elimination of the purification would be very advantageous by providing more rapid and cost effective analysis. As shown in Fig. 11, two identical PCRs using primes Nos. 1 and 3 was performed on the same HPV positive sample as described in Example 1. After the reaction, one of the amplified products was purified using purification kit (MP Biomedicals, LLC) and the other was not purified. Each of them was then assayed using the strip of the present invention as described in Example 6. As shown in Fig. 11, the signal from the unpurified product was lower than that of the purified.

To overcome the low signal from the unpurified products, the components in the PCR reaction which had caused the low signal was determined as follows. The same PCR reaction as above was performed in the presence of different chemicals in various amounts as described in Figs. 12 and 13. As shown in Fig. 12, it was found that the signal was most negatively affected by MgCl₂ at 2.5mM. Based on this result, the PCR amplified products were used unpurified instead treated with 50mM EDTA to remove MgCl₂ present in the reaction buffer before tested on the strip, which resulted in the same signal intensity obtained using the purified products (Fig. 13). In Fig 13, biotin and streptavidin were used for the detection.

### EXAMPLE 10

### Later flow assay with various types HPV positive samples

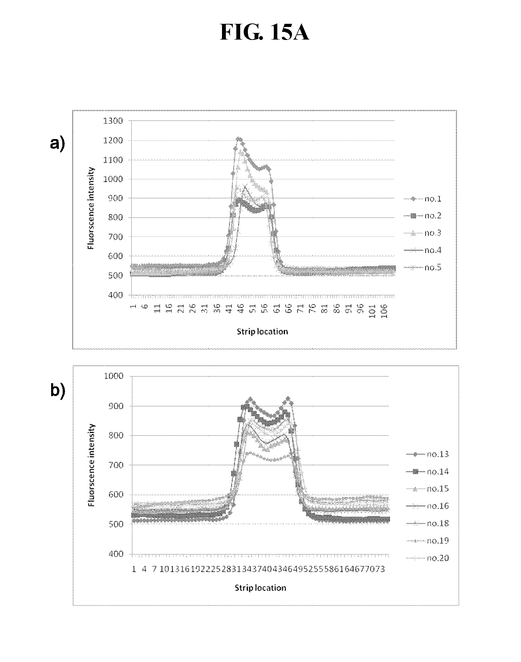

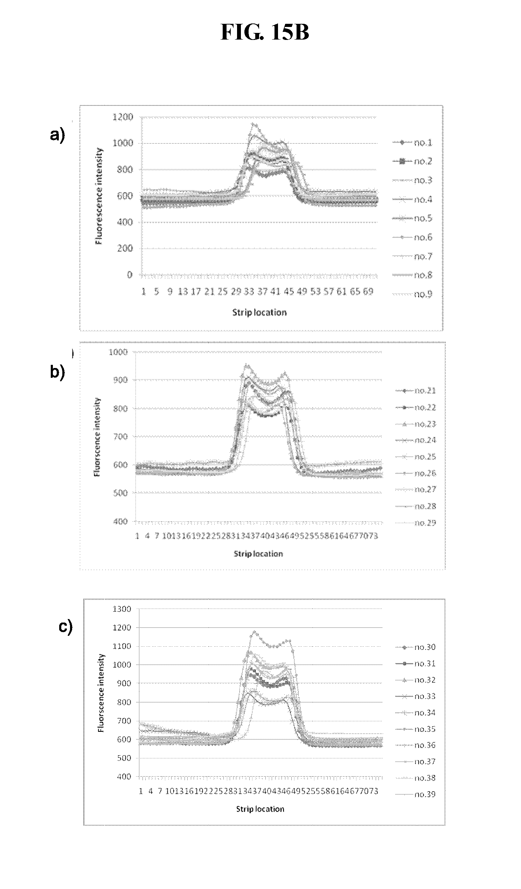

The genomic DNAs were obtained from each of 39 biopsy samples from cervical cancer patients with HPV positive (Table 3) (obtained from Yonsei University, informed consent was obtained from each of the patient) and PCR was performed for 30 cycles on each DNA sample using primes Nos. 1 and 3 as described in Example 1. The amplified products were confirmed by electrophoresis on a 2% agarose gel (Fig. 14) and assayed on the strip as described in Examples 4 and 6. The results are shown in Fig. 15 (A and B). As shown in Fig. 15, some sample identified HPV positive was not found to have HPV band on the electrophoresis assay, but all the sample identified with HPV positive generated fluorescent signal by the assay using the present strip.

**[Table 3]**

| **Cervical Cancer Lesion** | **#** |
|---|---|
| adenocarcinoma | 2 |
| CINI | 4 |
| CINII | 3 |
| CINIII | 5 |
| CIS | 9 |
| CNI | 5 |
| CSIL | 1 |
| HGSIL | 3 |
| invasive | 2 |
| LGSIL | 1 |
| Micro invasive | 2 |
| SCC | 2 |
| **Total** | **39** |

| | |
|---|---|
| CIN (Cervical intraepithelial neoplasia ), CIS (Carcinoma in situ), HGSIL (High grade squamous intraepithelial lesion), LGSIL (Low grade squamous intraepithelial lesion), SCC (Squamous Cell Carcinoma), CSIL (Cervical Squamous Intraepithelial Lesions) | |

Further, When the PCR was performed on the DNA extracted from the nonspecific bands of lane #37 (Fig. 14) under the same condition as above, no positive fluorescent signal was obtained (Fig. 16). This indicates that non-specifically amplified products do not interfere with HPV detection using the present system, and proves the excellent specificity of the present system. In Figs. 15 ad 16, non-specific indicates negative control.

### EXAMPLE 11

### Later flow assay with the PCR products amplified using the biopsy sample without prior DNA extraction

In general, to perform a PCR using primers labeled with Biotin, the DNA needs to be extracted and purified from the biopsy sample before being used as a template. However, in order to reduce sample preparation time and thus the total assay time required, the biopsy sample per se used as a template without prior DNA extraction. The PCR was done on the HPV positive sample No.5 of Example 10 under the same condition as described in Example 1. Then the amplified products were analyzed by electrophoresis and the later flow assay as described in Examples 4 and 6. As shown in Fig. 17, no positive result was obtained in the electrophoresis assay, which is in contrast to the result obtained from the later flow assay using the present system where positive fluorescent signal was obtained (Fig. 18). These results indicate that the present system can further reduce the total analysis time by eliminating the required DNA extraction without scarifying the specificity.

The conventional HPV test generally requires DNA preparation steps from biopsy samples and DNA amplification using PCR for the determination of cervical cancer. These conventional methods provide specificity and sensitivity to some degree; however they have disadvantage of long reaction time and complicated process involved. In contrast, the present system based on a DNA chromatography provides not only better specificity and sensitivity for the detection, but also provides reduced assay time due the elimination of DNA extraction before PCR and purification step after PCR and rapid chromatography reaction, which results in at least 50% reduction in the total assay time. In addition, it is contemplated that the present system is not limited to HPV detection and can be used for the detection of DNA from various origins together with various capture molecules. Also contemplated herein is the assay where more than one types of capture molecules are employed for the simultaneous detection/determination of multiple types of DNAs.

With respect to the use of substantially any plural and/or singular terms herein, those having skill in the art can translate from the plural to the singular and/or from the singular to the plural as is appropriate to the context and/or app lication. The various singular/plural permutations may be expressly set forth her ein for sake of clarity.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following

## Claims

1. A later flow strip for qualitative and/or quantitative determination of a nucleic acid in a sample comprising: a sample pad providing a site of sample application; an absorption pad; a chromatographic medium located between the sample pad and the absorption pad in a way to allow capillary flow communication with each other, the chromatographic medium containing at least one capture molecule which are capable of hybridizing sequence specifically to the nucleic acid in the sample which is applied to the sample pad; and a solid support supporting the chromatographic medium, the sample pad and the absorption pad which are located on the solid support in the same plane so as to permit capillary flow communication with each other.

2. The strip according to Claim 1, wherein the at least one capture molecule has a sequence derived from Human Papilloma Virus.

3. The strip according to Claim 1, wherein the at least one capture molecule has a sequence derived from HPV subtypes selected from the group consisting ofHPV subtypes 16, 18, 26, 31, 33, 35, 39, 45, 51, 52, 53, 56, 58, 59, 66, 68, 70, 73, 82, 6, 11, 40, 42, 43, 44, 54, 61, 72, 81 and CP108.

4. The strip according to Claim 3, wherein the HPV subtypes are selected from the group consisting of 6, 11, 16, 18, 31, 45 and 51.

5. The strip according to any one of Claims 1 to 4, wherein the nucleic acid which is applied to the sample pad is a product of polymerase chain reaction.

6. The strip according to Claim 5, wherein the PCR is performed for 1 to 10 cycles.

7. The strip according to Claim 6, wherein the PCR is performed for 1 to 5 cycles.

8. The strip according to any one of Claims 1 to 7, wherein the nucleic acid which is applied to the sample pad is labeled with a fluorescent material or a biotin.

9. A later flow strip for qualitative and/or quantitative determination of a nucleic acid in a sample comprising: a sample pad providing a site of sample application; an absorption pad; a chromatographic medium located between the sample pad and the absorption pad in a way to allow capillary flow communication with each other, the chromatographic medium containing at least one capture molecule which are capable of hybridizing sequence specifically to the nucleic acid in the sample which is applied to the sample pad; and a solid support supporting the chromatographic medium, the sample pad and the absorption pad which are located on the solid support in the same plane so as to permit capillary flow communication with each other, wherein the nucleic acid in the sample is labeled with a fluorescent material or a biotin and is a PCR product.

10. The strip according to Claim 9, wherein the PCR is performed for 1 to 10 cycles.

11. The strip according to Claim 5, wherein the PCR is performed for 1 to 5 cycles.

12. The strip according to any one of Claims 9 to 11, wherein the at least one capture molecule has a sequence derived from Human Papilloma Virus.

13. The strip according to Claims 9 to 11, wherein the at least one capture molecule has a sequence derived from HPV subtypes selected from the group consisting ofHPV subtypes 16, 18, 26, 31, 33, 35, 39, 45, 51, 52, 53, 56, 58, 59, 66, 68, 70, 73, 82, 6, 11, 40, 42, 43, 44, 54, 61, 72, 81 and CP108.

14. The strip according to Claim 13, wherein the HPV subtypes are selected from the group consisting of 6, 11, 16, 18, 31, 45 and 51.

15. A system comprising the strip according to any one of Claims 1 to 14 and an epifluorescence detection device for the detection of a signal emitted from a site where the capture molecule is present.

16. A method for qualitative and/or quantitative determination of a nucleic acid in a sample using any one of the strips according to any one of Claims 1 to 14, the method comprising the steps of: applying a liquid sample containing a nucleic acid sequence of interest labeled with a fluorescent or a biotin to a sample pad of the strip; allowing the liquid sample to flow through to an absorption pad of the strip, wherein at least one capture molecule immobilized on a particular location of the strip reacts sequence-specifically with the nucleic acid sequence of interest to form a hybrid; and detecting a signal emitted from the labels on the hybrid using an epifluorescence detection device, where the presence of the signal indicates the presence of the nucleic acid of interest or the signal is used to quantify the amount of the nucleic acid of interest in the sample.

17. The method according to Claim 16, wherein the at least one capture molecule has a sequence derived from Human Papilloma Virus.

18. The method according to Claim 16, wherein the at least one capture molecule has a sequence derived from HPV subtypes selected from the group consisting ofHPV subtypes 16, 18, 26, 31, 33, 35, 39, 45, 51, 52, 53, 56, 58, 59, 66, 68, 70, 73, 82, 6, 11, 40, 42, 43, 44, 54, 61, 72, 81 and CP108.

19. The method according to Claim 18, wherein the HPV subtypes are selected from the group consisting of 6, 11, 16, 18, 31, 45 and 51.
